# EUROPEAN PATENT SPECIFICATION

(11) **EP 2 859 013 B1**
(45) Date of publication and mention of the grant of the patent: **31.08.2016**
(21) Application number: 13803756.9
(22) Date of filing: 12.06.2013
(51) Int. Cl.: C07K 14/195, C07K 17/10, C07D 487/00

(54) **DIFFERENTIAL FUNCTIONALIZATION OF POLYMERS WITH AMINO-OXY REAGENTS FOR DIAGNOSTIC ASSAYS**
DIFFERENZIELLE FUNKTIONALISIERUNG VON POLYMEREN MIT AMINO-OXY-REAGENZIEN FÜR DIAGNOSTISCHE TESTS
FONCTIONNALISATION DIFFÉRENTIELLE DES POLYMÈRES AVEC DES RÉACTIFS AMINO OXY POUR DES DOSAGES DIAGNOSTIQUES

(30) Priority: 12.06.2012 US 201261658751 P
(43) Date of publication of application: 15.04.2015
(73) Proprietor: Fina Biosolutions LLC, Rockville, MD 20850 (US)
(72) Inventor: LEES, Andrew, Silver Spring, MD 20910 (US)
(74) Representative: Williams, Richard Andrew Norman
(86) International application number: PCT/US2013/045413
(87) International publication number: WO 2013/188539

(56) References cited:
- WO-A2-2005/072778
- WO-A2-2011/053971
- US-A- 5 543 332
- US-A1- 2005 169 941
- US-A1- 2007 031 371
- US-A1- 2010 273 994
- US-A1- 2012 214 187
- US-A1- 2013 302 877
- US-B1- 6 299 881

## Description

### Background

### 1. Field of the Invention

This invention is directed to the synthesis of functionalized polymers for diagnostic assays and detection methods. In particular, polymers of the invention are differentially functionalized at a terminal functional group and along the repeat units of the polymer chain.

### 2. Description of the Background

Assay systems are used in many different fields including, but not limited to medicine for the detection of diseases and disorders including cancers, infections, and genetic mutations. In general, an assay system comprises compositions and methods of detection, which provides specificity for the assay, and a means of signaling that detection which provides a readout or indication. Assays systems typically contain a chemical or macromolecular component of a known specificity.

A great many diagnostic assays utilize antibodies to generate specificity such as the ELISA assay. Methods of performing ELISAs are well known in the art and a variety of formats are currently utilized. Methods of setting up ELISAs are described, for example, in Elisa: Theory and Practice (Methods in Molecular Biology) by John R. Crowther. Humana Press, 1995; Immunoassays: A Practical Approach (Practical Approach Series) James P. Gosling, and Assay Development: Fundamentals and Practice. Ge Wu., John Wiley & Sons, 2010. Other antibody-based assay systems are lateral flow devices. These are described in Lateral Flow Immunoassay, Editors: R. Wong & H. Tse, Humana Press, 2009.

A variety of formats can be used for setting up antibody-based detection systems. In a typical system, a detection antibody is linked to an enzyme, such as horseradish peroxidase. In another format, the detection antibody may be biotinylated and the signal component a streptavidin-enzyme complex. Although ELISA is proved over and over again to be commercially successful, the basic process involves individual components that recognize and bind to each other in a successive process. Increased affinity is dependent on the affinity of each of the individual components as well as the ability of the signal to be read. A need exists for increased signaling which would greatly enhance the usefulness of this already commercially commanding procedure. WO-A2-2005072778 discloses polymers with a single amino-oxy functionalization for the manufacture of conjugates.

### Summary of the Invention

The present invention overcomes the problems and disadvantages associated with current strategies and designs, and provides new tools and methods for the differential functionalization of a carbohydrate polymer, with one functional group on the end of the polymer and different functional groups along the length of the polymer chain.

One embodiment of the invention is directed to methods for the differential functionalizing a polymer. These methods comprise functionalizing a first moiety of the polymer with a functional reagent such that the polymer contains at least one functional group and functionalizing a second moiety of the polymer containing the at least one functional group by reaction with an amino-oxy reagent to form at least one different functional group. Preferably the first moiety is the polymer chain of a polysaccharide, an oligosaccharide, a carbohydrate or a carbohydrate-containing molecule and also preferably, the at least one functional group is an amine, a carboxyl, a thiol, or an amino-oxy group. The method may preferably comprise wherein the at least one functional group is a single functional group and also preferably, the single functional group is an amine, a carboxyl, a thiol or an amino-oxy group. Preferably the the first moiety is a dextran polymer and the second moiety is the reducing end of a polymer chain, a polysaccharide, an oligosaccharide, a carbohydrate or a carbohydrate containing molecule wherein the at least one different functional group is an amine, a carboxyl, a thiol, hydrazide, hydrazine or an amino-oxy group. Also preferably, the at least one different functional group is a single functional group and the single functional group is an amine, a carboxyl, a thiol or an amino-oxy group. Preferably the reaction with the first functional group precedes functionalizing the reducing end of the polymer by reaction with an amino-oxy reagent and the method is performed in an aqueous medium.

Another embodiment of the invention comprises methods of differentially functionalizing a polymer comprising functionalizing a first moiety of the polymer by reaction with an amino-oxy reagent to form at least one functional group and functionalizing a second moiety of the functionalized polymer by reaction with a functional reagent to form at least one different functional group. Preferably the first moiety is a reducing end of the polymer wherein the polymer comprises a polysaccharide, an oligosaccharide, a carbohydrate or a carbohydrate containing molecule. Preferably the at least one functional group comprises an amine, a carboxyl, a thiol, hydrazide, hydrazine or an amino-oxy group, or the at least one functional group is a single functional group such as, for example, an amine, a carboxyl, a thiol, hydrazide, hydrazine or an amino-oxy group. Preferably the first moiety is a reducing end of a dextran polymer, the second moiety is the polymer chain of a polysaccharide, an oligosaccharide, a carbohydrate or a carbohydrate containing molecule, and the at least one functional group is an amine, a carboxyle, a thiol, hydrazide, hydrazine or an amino-oxy group. Preferably the at least one different functional group is a single functional group such as, for example, an amine, a carboxyl\ a thiol, hydrazide, hydrazine or an amino-oxy group. Preferably the second moiety is a dextran polymer. Also preferably, the functionalizing the reducing end of the polymer by reaction with an amino-oxy reagent precedes functionalizing precedes the reaction with the second functional group and is performed in an aqueous medium.

Another embodiment of the invention is directed to methods of differentially functionalizing a polymer comprising functionalizing the polymer with an amino oxy reagent to react with a carbonyl group at one terminus of the polymer; and reacting the functionalized polymer with a reagent that binds to the polymer exclusive of the terminus. Alternatively, the method of differentially functionalizing a polymer may comprise, in order, reacting the polymer with a reagent that binds to the polymer exclusive of the terminus; and functionalizing the polymer with an amino oxy reagent to react with a carbonyl group at one terminus of the polymer. Preferably, one or more reaction conditions for functionalizing the polymer with an amino-oxy reagent are different from one or more reaction conditions for reacting the functionalized polymer with a reagent. Reaction conditions include, but are not limited to reaction time, reaction temperature, and the presence or absence of reaction components. Preferably the polymer is dextran and the carbonyl group is an aldehyde, wherein functionalized polymer bound with the reagent is selected from the group consisting of monoamine dextran, monobiotin dextran, monobiotin HRP-dextran, monoamine-carboxyl dextran, monothiol-carboxyl dextran, and monothiol amino dextran.

Another embodiment of the invention is directed to methods of differentially functionalizing a polymer comprising functionalizing a first moiety of the polymer with reagent A such that the polymer contains at least one functional group A; and functionalizing a second moiety of the polymer containing the at least one functional group A by reaction with a second reagent B to form at least one different functional group B, wherein group A and group B are different, the polymer contains one or more of group A or group B and one of Group A or B, and reagent A is an aminooxy-containing reagent if A is one, or reagent B is an aminooxy-containing reagent if B is one. Preferably the polymer is dextran

Another embodiment of the invention is directed to differentially functionalized polymers created by the method of the invention. Preferably the differentially functionalized polymers include monoamine dextran, monobiotin dextran, monobiotin HRP-dextran, monoamine-carboxyl dextran, monothiol-carboxyl dextran, and monothiol amino dextran.

Other embodiments and advantages of the invention are set forth in part in the description, which follows, and in part, may be obvious from this description, or may be learned from the practice of the invention.

### Description of the Figures

Figure 1 Chart of area of streptavidin HPLC SEC peak vs. mole biotin-(Flu Dex)/mole streptavidin.
Figure 2 Results of an ELISA showing horseradish peroxidase polymer conjugates with a monobiotin terminal functional group binding to streptavidin adsorbed to the ELISA plate.
Figure 3 Graph of retention time of mono-biotin-(HRP-dextran) with streptavidin molar ratio additions.
Figure 4 ELISA of mono-biotin-(HRP-dextran) conjugates with varying rations of polymer:streptavidin (SA).
Figure 5 Charts of fraction of biotin-antibody plus SA dextran.
Figute 6 Chromatogram of Affinity purified goat-anti-mouse IgG antibody functionalized with maleimide using GMBS and dialyzed into PBS-EDTA showing a reduction in the area of antibody peak and a shift to higher molecular weight complexes.
Figure 7 Antibody-SBP/Dextran complex added to a mouse IgG coated ELISA plate shows a working 4-parameter logistic curve.

### Description of the Invention

It has been surprisingly discovered that polymers can be conveniently functionalizing using commercially available components and with steps that are performed for reduced times and under reduced temperatures, as compared with other conventional methods, and in an aqueous medium. Importantly, the invention allows for the functionalization of the reducing end of the polymer and differential functionalization of the regions within the length of the polymer. Preferably, the invention provides a method for preparing polymers with a different functional groups or functionalities along the repeat units of the polymer chain than on the reducing end.

By functionalization, it is preferred that the addition of a chemical group is to be conveniently further modified. For example, the hydroxyls of a carbohydrate are not easily modified. By functionalizing them with amino groups or carboxyl groups many additional reactions can be conveniently carried out under mild conditions using commercially available reagents (e.g., reduced temperature and times as compared to conventional procedures). Functionalization can also mean the addition of molecules that have function or usefulness, for example, in diagnostic applications. Preferred examples include the addition to the polymer of a biotin molecule, enzymes such as horseradish peroxidase, molecules of clinical relevance such as anti-cancer drugs or imaging agents, or other functionally useful moieties.

In certain preferred embodiments, functionalization is the addition of a chemical group with new functionality or the addition of a molecule, protein, oligonucleotide, and the like, that provides a desirable function. The invention provides for functionalizing the end of the polymer and differentially functionalizing the repeat units of the polymer chain.

The invention allows for different molecules (e.g., proteins, dyes, binding agents, and the like) on the reducing end than along the repeat units of the polymer chain exclusive of the terminus. As embodied and broadly described herein, the present invention is directed to the functionalization of a carbohydrate polymer.

It has also been surprisingly discovered that the dextran polymer chain can be functionalized while leaving the reducing end available for functionalization with an aminooxy reagent. As illustrated in the examples, the invention allows the dextran polymer to be conveniently modified so that there is one functional group on the end and functional groups with different reactivity along the polymer chain. Another surprising finding is that a reagent containing both an aminooxy group and an amine (e.g., aminooxy ethylamine) which could react at either the amine or the aminooxy group will preferentially react with the reducing end of the dextran polymer to give an oxime, so that a product with a free amine is formed.

Catalysts, such as, preferably, analine and related compounds, can be used to promote the reaction of the aminooxy group with carbonyls (Dawson et a.l Angew Chem Int Ed Engl. 2006 45(45):7581-4.). Although dextran polymers containing different functional groups on the end and along the polymer chain have been described (Mann et al, Bioconjug Chem. 1992 3(2):154-9), they are prepared using a more difficult process and use hydrazone chemistry, not oxime linkages. Hydrazones are not as stable as oximes, the reaction is not as efficient and require harsher conditions to drive to completion. The methods described in this invention have the advantage of being easier, faster and more straightforward as compared to conventional procedures. Furthermore the preparation can be done in aqueous media, although it may optionally be performed in part or complete organic solvents. The dextran size may vary from monomer up to molecular weights up to greater than 2000 kDa.

Lees (U.S. Patent Application Publication No. 2005/0169941; Vaccine 2006 Feb 6;24(6):716-29) describes the use aminooxy reagents for the functionalization of carbohydrate polymers for use as conjugate vaccines, including functionalization of the reducing end of a dextran polymer. The methods described and detailed herein allow for the functionalization of carbohydrate polymers with different functional groups on the end and the length of the polymer.

Another example of functionalizing the reducing end of dextran polymers is given in Chem Commun (Camb). 2012 Apr 18;48(31):3781-3. Epub 2012 Mar 9.Synthesis of polysaccharide-b-PEG block copolymers by oxime click. Novoa-Carballal R, Müller AH. Not described therein is functionalizing both the end and the repeat units of the polymer chain. Protein is linked only to the reducing end of dextran polymers (Bioconjug Chem. 1997 Nov-Dec;8(6):927-34).

A preferred aspect to the facile production of monofunctionalized dextran with different functionality along the chain is to employ an aminooxy reagent to functionalize the reducing end of the dextran polymer and an orthogonal chemistry to functionalize the polymer chain. Selection of suitable orthoganol chemistry allows for the reducing end to be functionalized either prior or subsequent to the functionalization of the dextran polymer chain. Similarly, the dextran polymer chain may first be functionalized and the reducing end of the polymer reacted with an aminooxy reagent.

As described and detailed herein, bifunctional aminooxy reagents facilitate functionalization of a carbonyl group on the terminal end of the polymer. Aminooxy acetate (available from Sigma Aldrich), allows for the conversion of an aldehyde to a carboxyl group while aminooxy ethylamine (available from Activate Scientific GMBH) converts an aldehyde to an amino group. Diaminooxyhexane (available from Research Organics) allows for the conversion of an aldehyde to an aminooxy group. Many reactions are known for the further reactions of amines and carboxyl groups (Bioconjugate Techniques, GT Hermanson Academic Press 2nd ed, 2008). Furthermore, a number of methods are available to protect amines and carboxyls to allow modification of the polymer chain.

A variety of methods have been described to functionalize the dextran polymer chain (Inman, J Immunol. 1975,114(2 Pt 1):704-9.), including preferably chloroacetylation, oxidation, bromoallyl groups, epichlorhydrin, cyanuric acid (J Pharm Sci. 1989 Feb;78(2):117-21) and others. Surprisingly, it was found that dextran can be activated with CDAP and functionalized along the polymer chain while still leaving the reducing end available for reaction with an aminooxy reagent.

Antibodies have great value as detection reagents in diagnostic assays. Signal molecules (e.g., fluorescent molecules, biotin, enzymes) are often attached to antibodies that can also be used to target drugs and imaging agents. However, there are generally limits to the extent of modification that can be done and care must be taken not to damage the antibody during any labeling process. Very often, over labeling or functionalization of the antibody decreases its effectiveness. A valuable application of the method described herein includes the ability to minimally modify the antibody while gaining the benefits of linking a large number of useful molecules. For example, in a preferred method, a limited number of polymers can be attached to the antibody, thereby minimizing antibody modifications. Instead, it is the polymer which carries the molecules to be directed by the antibody. The polymer may carry, preferably, fluorescent or chemiluminesent molecules. The polymer can also be functionalized with enzymes such as horseradish peroxidase, soybean peroxidase, alkaline phosphatase or catalase. The polymers can also be functionalized with MRI imaging agents or anti-cancer drugs, allowing the antibody to direct the imaging agent or drug to the desired site, in vivo.

The following examples illustrate embodiments of the invention, but should not be viewed as limiting the scope of the invention.

### Examples

### Example 1

### Dextran polymers functionalized on the reducing end with a single functional group.

A general protocol for labeling the end of the dextran polymer is as follows: Dextran polymer is solubilized at 25 mg/ml in an aqueous buffer of about pH 5 and an excess of the reagent 2-(Aminoxy)ethanamine dihydrochloride (AOEA) (Activate Scientific GMBH) is added. Typically 5-50 fold molar excess over the polymer is used. The solution is maintained at about pH 5 at 70°C for 24 hrs and then buffer exchanged by a combination of desalting on a G-25 gel filtration column (equilibrated with saline) and dialysis against saline. The product is a monoamine dextran. When aminooxy acetate is substituted for aminooxy ethanamine in the above reaction, the product is a monocarboxyl dextran. When diaminooxy cysteamine disulfide is substituted for aminooxy ethylamine in the above reaction, following reduction, the product is a monothiol dextran. When excess diaminooxy hexane is substituted for aminooxy ethylamine in the above reaction, following reduction, the product is a monoaminooxy dextran. These monofunctionalized dextrans can be further modified using well-known methods (Bioconjugate Techniques, Hermanson). Many other bifunctional aminooxy reagents can be used in addition to the ones named.

By way of example, mono-amino dextran was prepared using 250mg of 70kDa molecular weight dextran (Pharmacosmos A/S, Denmark) in 2.5mL of 0.1M sodium acetate buffer at pH 5.0 resulting in a concentration of 100mg/mL or 1.43mM dextran. The amine reagent 2-(Aminoxy)ethylamine dihydrochloride (AOEA) (Activate Scientific GMBH) was added to a 12-fold molar excess (17.2mM) or 12.8mg. The sample was adjusted to pH 5.7 by titration with 10µL aliquots of 5M NaOH.

The sample was capped and heated on a water bath at 73°C for 72 hours (although shorter times may also be used). A 50mg aliquot (0.5mL) was removed and desalted on a XK16 column containing 15ml of G-25 Sephadex. HPLC analysis determined the dextran concentration of the washed fraction to be 4.84mg/mL or 69.1µM. The amine concentration of this fraction was quantified using the 2,4,6-Trinitrobenzene Sulfuric acid (TNBS) assay for free amines and was determined to be 89µM. The amine:dextran ratio was calculated to be 1.3 mole NH₂ per mole 70 kDa dextran.

The dextran functionalized with a monoamine can then be reacted, for example, with fluorescein isothiocyantate (FITC) to make a dextran polymer with a single fluorescein. Such fluorescent polymers can be used as neuronal tracers. The monoamine dextran can be modified with thiol groups, for example, with SPDP. The monothiol dextran can be adsorbed to gold surfaces via the thiol and thereafter used as passivating agents.

The monoamine dextran can be linked to biologically relevant molecules to decrease immunogenicity in the same manner that pegylation is used to reduce immunogenicity. As dextran is very hydrophilic, the monoamine dextran can also be used to modify proteins or surfaces to make them less hydrophobic which is less adhesive or sticky to certain surfaces and other molecules.

### Example 2

### Functionalization of the polymer chain prior to functionalization of the reducing end.

Dextran is carboxymethylated using chloroacetic acid in 3 M NaOH (Inman 1975) producing a carboxymethyl dextran polymer (CMdex). It was surprisingly found that the reducing end of the dextran polymer could still be functionalized with aminooxy reagent, as described in Example 1.

Mono-amino-(CMdextran) was prepared as follows. 500 mg of 70kDa molecular weight CM dextran was solubilized in 3.0 mL of water. The sample was adjusted to pH 5.2 by the addition of 170µL of 3M sodium acetate buffer. Water was added to bring the total volume to 5.0mL. The resulting CM-dextran concentration was 100mg/mL or 1.43mM.

The aminooxy amine reagent, 2-(Aminoxy)ethanamine dihydrochloride (AOEA), was added to a 12-fold molar excess (17.2mM) or 12.8mg. The sample was adjusted to pH 5.7 by titration with 10µL aliquots of 5M NaOH.

The sample was capped and heated on a water bath at 73°C for 21 hours. A 50mg aliquot (0.5mL) was removed and desalted on a XK16 column containing 15ml of G-25 Sephadex. A 5.0mL aliquot of the void volume eluant containing the polymer was washed twice with 10mL of saline on an Amicon Ultra 15 spin device centrifugal filter (10kDa MWCO). HPLC analysis determined the dextran concentration of the washed fraction to be 2.56mg/mL or 36.6µM. The amine concentration of this fraction was quantified using the 2,4,6-Trinitrobenzene Sulfuric acid (TNBS) assay for free amines and was determined to be 83µM. The amine:dextran ratio was calculated to be 1.2 NH₂ per mole of 70kDa CMdextran.

Examples of applications of monofunctional-(derivatized dextrans). The monoamine-(CMdextran) was biotinylated to produce monobiotin-(CMdex) as follows. 5 ml of 40 mg/ml monoamino dextran (70kDa) was combined with 0.5 ml of 1 M HEPES pH 8 and 6.7 mg of S-NHS LC biotin (Pierce product # 21335) was added as a solid while vortexing. The reaction was allowed to proceed overnight at 4°C and then extensively dialyzed into saline. This product is monobiotin-(CMdex)

The fluorescein hydrazide was added to the carboxyl groups on the monobiotin CM dextran as follows: 0.66 ml of 15 mg/ml monobiotin-(CMdex) produced above was combined with 100 ul of 1 M MES pH 5 buffer and 40 ul of a 100 mg/ml solution of EDC was added, followed by the addition of 1.9 mg Fluorescein semicarbazide (Molecular Probes #F-121) added. A precipitate formed on addition. The reaction was allowed to proceed overnight. The pH was raised to 9, which resulted in a clear solution. Free fluorescein was removed by desalting on a G25 column (1.6x15 cm), equilibrated with 0.1 M sodium borate, pH 9. The void volume was pooled and dialyzed into saline. The product is monobiotin -(FLU-Dex).

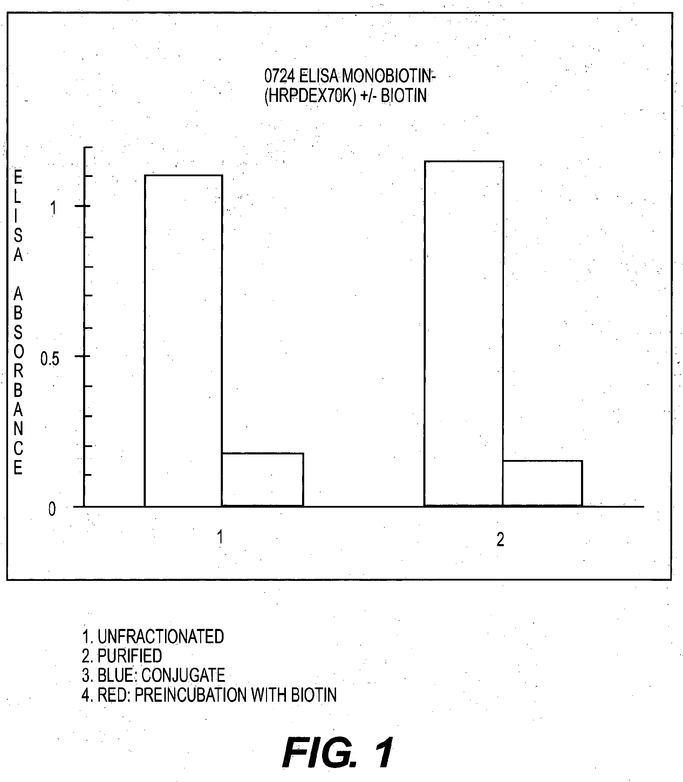

The following experiment was performed to demonstrate that the product contained a single biotin. Streptavidin has four biotin binding sites. Streptavidin was incubated with 0-4 moles of monobiotin -(FLU -Dex) and the complex analyzed by size exclusion chromatography. The area of the starting streptavidin peak was plotted against the polymer:streptavidin molar ratio added. At a ratio of 4:1, the streptavidin peak area is about zero, as binding converts it to a high molecular weight conjugate (Figure 1). If there was more or less than one biotin per polymer than it would take less or more polymer, respectively, for the streptavidin to be converted. Therefore, these results demonstrate that there was, on average, one biotin per polymer.

Alternatively, the monobiotin-CM dextran is converted to monobiotin-(amino-dextran) using carbodiimide activation (EDC) and ethylene diamine to produce monobiotin-(aminodextran). This reaction is carried out as described in Inman, (Journal of Imm. 114:704, 1975). This monobiotin-(amino-dextran) polymer can be linked via the amino groups to HRP or amino-HRP using known methods (e.g.,Brunswick et al. J Imm 140:3364,1988; Bioconjugate Techniques, Hermanson) and used, for example, in an ELISA, but with detection of the enzyme product.

The terminal amine of the monoamino-(CMdextran) can be functionalialized with an appropriate oligonucleotide and then labeled on its carboxyl groups with, for example, fluorescent groups, enzymes or various dyes.

Monobiotin-(CM Dex) or monobiotin-(AminoDex) can also be functionalized along the polymer chain with quantum dyes, lanthanide dyes, chemiluminescent and luminescent dyes. This process creates a polymer chain with multiple labels along the chain that can be linked at the polymer end to another molecule, such as streptavidin or an antibody. These reagents are also useful in fluorescent cell assays, cell sorters, FACScan, histology, immunohistology and the like.

### Example 3

The dextran polymer is first functionalized at its reducing end with an aminooxy reagent as shown in Example 1 is optionally protected. An orthoganol chemical reaction is then used to functionalize the dextran polymer chain.

### Example 4

Monoamino-(CM dextran) is prepared as described in Example 2. The monoamine is then reacted with an NHS ester thiol ester (such as SATA). The carboxyl groups along the polymer chain are converted to amino groups using carbodiimide and ethylene diamine (Inman, 1975). This creates a polymer with a monothioester on the end and amino groups along the polymer chain. The amino groups subsequently reacted with the thiolating reagent SPDP. The product is a polymer with a thioester at the reducing end and thio-pyridyl groups along the polymer chain. The thioester is deprotected with hydroxylamine, resulting in a free thiol on the end of the polymer. Hydroxylamine does not affect the thio-pyndyl groups.

An antibody of interest is derivatized with maleimide (e.g., using GMBS) and reacted with the thiol tipped thio-pyridyl polymer. The thio-pyridyl groups along the polymer chain are then deprotected using DTT, revealing thiol groups which are subsequently reacted with maleimide-derivatized horseradish peroxide. The result is an antibody containing a limited number of polymers with many copies of HRP, without producing cross linked antibody or excessively modifying the antibody. Unconjugated antibody and HRP are removed by size exclusion chromatography.

### Example 5

### Use of antibody to direct an imaging agent to a site of interest.

Preparation of Monobiotin AmDex is as follows: An appropriate chelating reagent is reacted with the amino groups (e.g., DTPA), followed by Gd+3. The reaction of DTPA with amino-dextran is described in BBRC, 77(2) 581, 1977. In one example of the use of this monobiotin DTPA dextran is as follows: Biotinylated antibody directed against a tumor is administered, followed by streptavidin, which binds the biotinylated antibody and monobiotin Gd⁺³ polymer added. The Gd⁺³ is imaged by MRI. Similarly, the monobiotin polymer can be prepared with an anti-cancer drug and a similar system used to direct the drug to a tumor.

### Example 6

Monoamine dextran is prepared as in Example 1. The monoamine dextran is oxidized with sodium periodate, creating monoamine dextran with aldehydes along the chain. This polymer is then reacted with aminooxy acetate. The oxime bonds can optionally be reduced. The product is a monoamine-carboxy dextran.

### Example 7

Monocarboxyl dextran is prepared as in Example 1 by reaction of the reducing sugar with aminooxy acetate. The moncarboxyl dextran is then oxidized and the aldehydes produced then reacted with aminooxy ethy-amine to form oximes. The product contains a monocarboxyl group at the end and amino groups along the polymer chain. This product is monocarboxyl-(Aminodextran). Optionally the oxime bonds can be reduced.

### Example 8

Dextran is activated with CDAP and then reacted with hexane diamine. The reducing end of the aminodextran polymer is then reacted with aminooxy acetate to produce a polymer with a carboxyl on the end and amino groups along the chain. The product is monocarboxyl-(aminodextran). The amino groups can be coupled to proteins, fluorescent molecules, and the like using well known methods (e.g., Bioconjugate Techniques, Hermanson).

### Example 9

### Monobiotin-(HRP-Dextran70kDa)

Monobiotin dextran70 kDa dextran was prepared as in Example 1. The monobiotin dextran was buffer exchanged into saline. CDAP was added at a ratio of 1 mg CDAP/mg dextran, the pH was then maintained at 9 for 2.5 min and aminated-HRP (U.S. Patent Application Publication No. 2012/0214187) was added at 1 mg HRP/mg dextran and the reaction allowed to proceed overnight. One portion was fractionated on a Superdex200 column (GE Healthcare) to remove unconjugated HRP. Another portion was not fractionated but was dialyzed to remove low molecular weight reagents. The product was monobiotin-(HRP-dextran).

The ability of these monobiotin-(HRP-dextran) conjugates to bind streptavidin was evaluated by ELISA. Streptavidin was coated at 1 ug/ml onto ELISA plates. To evaluate, nonspecific binding, control wells were preincubated with biotin at 1 ug/ml to block biotin binding sites on the streptavidin.

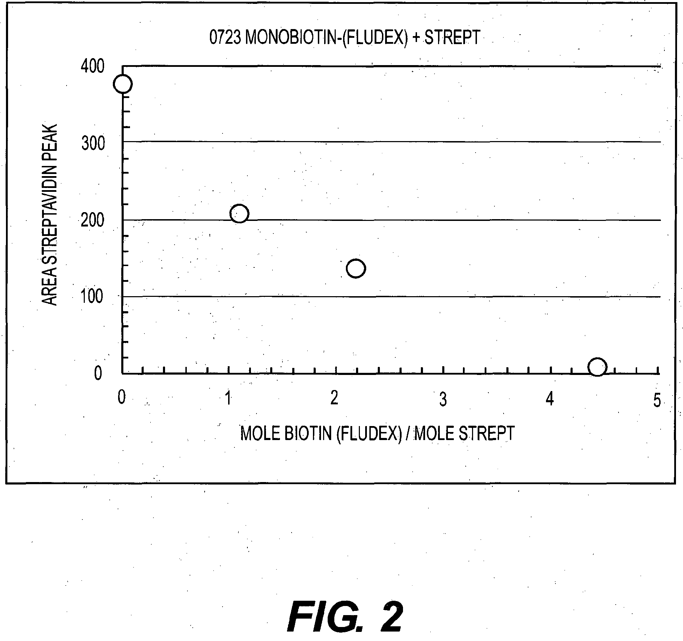

The results in Figure 2 indicate that the conjugates contain biotin, as binding to streptavidin is blocked by pretreatment with free biotin. These results also indicate that purification of the conjugate was not necessary, as the results were the same without removal of the unconjugated reagent.

### Example 10

### Preparation of a mono azido-dextran polymer for use as a "Click" reagent.

Monoamino dextran is prepared and reacted with an NHS ester of an azide (e.g., Quanta BioDesign #10503, Azido-dPEG®8-NHS ester). Amino HRP is then linked to the polymer using CDAP chemistry as in Example 9. The monoazido HRP dextran polymer is then linked to an antibody functionalized with an acetylene group using click chemistry. Suitable click reagents for functionalizing antibodies are available from Life Technologies.

### Example 11

### Preparation of multifunctionalized Dextran.

### Monoamino Dextran

An aqueous 100mg/mL dextran (70kDa) solution was adjusted to pH 5.5 and reacted with 10-fold molar excess 2-(Aminooxy)ethylamine dihydrochloride at 73°C for 72 hours, then reduced with 50-fold molar excess sodium borohydride. To remove reagent, the dextran was dialyzed against 0.5M NaCl followed by deionized water and then lyophilized. Solubilized product (monoamino dextran) was assayed for amines using TNBS and dextran using a resorcinol/sulfuric acid assay (Monsigny et al. J Analytical Biochem. 175:525, 1988). A ratio of 0.97 mole amine per mole of 70 kDa dextran was determined.

### Monobiotin Dextran

NHS-LC-Biotin (Molecular Biosciences) was added to the monoamino dextran in 5x molar excess and incubated for 1.5h at room temperature pH 7.4, followed by dialysis into DI water.

### Monobiotin-(HRP-dextran)

The monobiotin dextran polymer was activated with CDAP (U.S. Patent No. 5,651,971) and linked to amine-modified horse radish peroxidase (HRP-NH₂) at a 1 mg HRP/mg dextran ratio, as described in Example 9. After an overnight incubation, the reaction was quenched with 1M glycine and purified on a Superdex300 (GE Healthcare) column equilibrated with PBS. The middle of the high molecular weight peak was pooled and concentrated, and the A₂₈₀ and A₄₀₅ readings were taken to determine that the HRP concentration was 13mg/mL.

### Streptavidin + monobiotin-(HRP- Dextran)

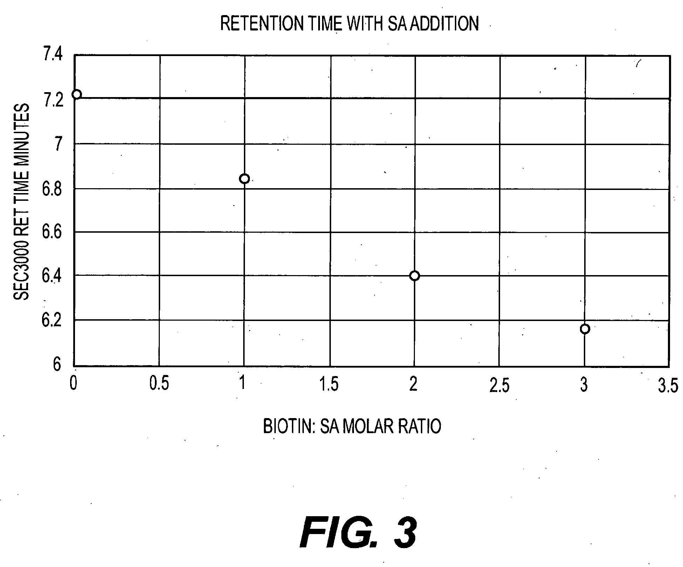

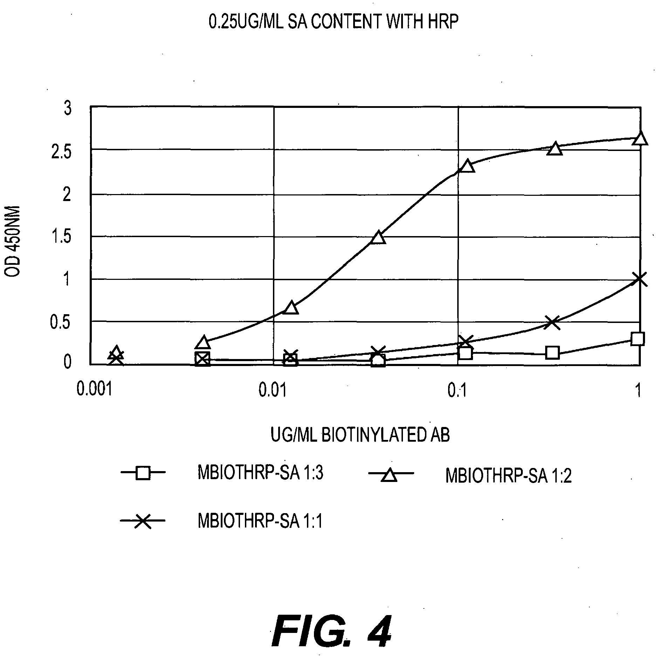

Streptavidin (ProZyme) was added to the mono-biotin-HRP dextran at a ratio of 1:1, 1:2, and 1:3 mole streptavidin/mole polymer and incubated overnight. Size exclusion HPLC showed no free streptavidin and gradual growth in size with an increasing amount of biotin-(HRP-dextran), consistent with the formation of complexes containing more polymer (Figure 3). An ELISA plate was coated with biotinylated antibody serially diluted down and the three conjugates were added at 250ng/mL streptavidin content. The results showed a 1:2 ratio of streptavidin to mono-biotin-. (HRP-dextran) performed best, indicating too little signal with the 1:1 ratio and not enough binding sites and/or steric hindrance with the 1:3 ratio (Figure 4).

### Example 12

### Preparation of Monoamino-Carboxyl Dextran

Carboxymethyl functionalized 70 kDa dextran was treated with 2-(Aminoxy)ethylamine dihydrochloride as described in Example 11. After dialysis against 0.25M NaCl the amine to dextran polymer ratio was found to be a 1:1 molar ratio.

### Monothiol-carboxyl dextran

N -Succinimidyl 3-(2-pyridyldithio)-propionate (SPDP), was added to the dextran in 5 molar excess and incubated for one hour at pH 8 followed by buffer exchange into 0.1M MES buffer, to yield a carboxymethyldextran polymer with a single protected thiol, mono thiopyridyl-(CMdextran).

### Monothiol amino dextran

The carboxyls along the polymer chain were converted to amino groups, without affecting the thio-pyridyl group on the end of the dextran polymer. A final concentration of 0.5M ethylenediamine·2HCl and 50mg/mL of 1-Ethyl-3-(3-dimethylaminopropyl)carbodiimide (EDC) was added to the solution. After a 3 hr incubation the solution was desalted using a G25 Sephadex column (GE Healthcare), equilibrated with PBS, 5 mM EDTA, pH 6.8. Hydroxylamine (1 M stock, pH 5) (not a necessary step) and DTT (1 M PBS/EDTA) was added to 25mM final concentration and incubated for 30 minutes. The functionalized dextran polymer was desalted again on the same column and fractions were assayed for dextran (resorcinol), amine (TNBS), and thiol (DTNB). Concentrations were 1.2mg/mL dextran (17µM), 786µM amine, and 18µM thiol for a 1:46:1 ratio. The 70 kDa dextran polymer contained 1 thiol group and 46 amines. Monothiol (amino dextran) (1 thiol, 46 amines/70kDa dextran).

### Preparation of amino dextran monothiol-Streptavidin

Streptavidin was functionalized with maleimide using GMBS and dialyzed into PBS-EDTA after 1 hr incubation. The maleimide-streptavidin was then added to the mono-thiol amino-dextran at equal molar ratios of streptavidin to monothiol-(aminodextran) polymer. The streptavidin-(amino dextran) conjugate was then fractionated on a 1 x 30 cm Superdex 200 column (GE Healthcare) size exclusion column to remove unconjugated streptavidin. The column was run at 0.5 ml/min, collecting 1 mL fractions, Fractions which eluted earlier than streptavidin alone, were the endlinked Streptavidin (amino-dextran) polymer.

### Preparation of amino dextran-monostreptavidin-antibody

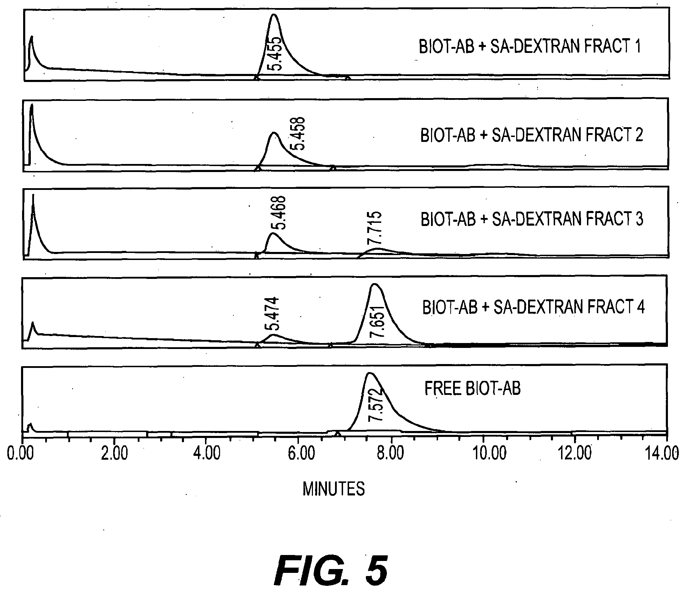

Biotinylated antibody was added to the Superdex 200 eluant fractions of streptavidin-(amino dextran) polymer. After a 1 hr incubation, the biotin-antibody + eluant fractions were analyzed by SEC HPLC using a Bio-SEP-S 3000 (Phenomenex) size exclusion column. Figure 5 shows the size exclusion chromatograms of the complexes. A higher molecular weight complex was observed in fractions containing the streptavidin dextran polymer indicating that the amino-dextran- streptavidin had bound to the biotinylated antibody.

### Example 13

Monothiol(protected)-(aminodextran) is reacted with NHS-fluorescein, an imaging agent (e.g., a gadolinium complex or an anti-cancer drug). The thiol is deprotected with DTT and the polymer dialyzed. The monothiol -(fluorescein (or otherwise derivatized)- dextran) is reacted with maleimide labeled antibody and purified. The resulting product is a fluorescent polymer, a polymer with imaging agents or a polymer with anti-cancer drugs end linked to the antibody.

### Example 14

### Modification of polymer end following conjugation of protein to polymer chain.

Amine modified soybean peroxidase (AmSBP) was linked to a 100kDa dextran chain with CDAP chemistry and fractionated to remove unconjugated AmSBP, using am S300HR column (1.6 x 55 cm) (GE Healthcare), equilibrated with 0.2M acetate buffer pH 5.5. Bis-(aminooxyacetamido)cystamine (Solulink) was incubated with SBP-dextran polymer at pH 5.5 (45°C 5 days). After stabilizing the oxime with sodium borohydride, the disulfide was reduced with DTT to give mono-thiol (SBP-dextran). DTNB assay for thiols gave 185µM free thiol while the resorcinoal assay for dextran gave 180µM dextran. Thus there was 1 thiol per SBP-dextran polymer. SBP concentration was determined to be 0.7mg/mL or 18.4µM by A₄₀₅ and A₂₈₀. The product was monothiol-(SBP-dextran).

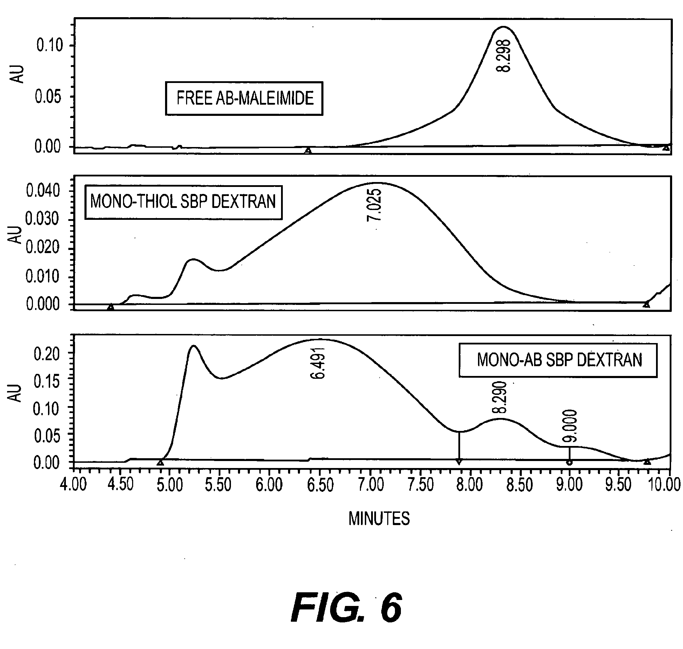

Affinity purified goat-anti-mouse IgG(H+L) antibody (Equitech-Bio, Inc) was maleimide functionalized using GMBS and dialyzed into PBS-EDTA buffer. Maleimide-antibody was added to the monothiol (SBP-dextran) at a 1:6 molar ratio. After 2 hrs of incubation the reaction mixture was analyzed by SEC HPLC using a Tosoh G4000 SEC column. The chromatogram (Figure 6) shows a marked reduction in the area of the antibody peak and a shift to higher molecular weight complexes (earlier elution time).

### ELISA assay using goat-anti-mouse IgG-(SBP-dextran)

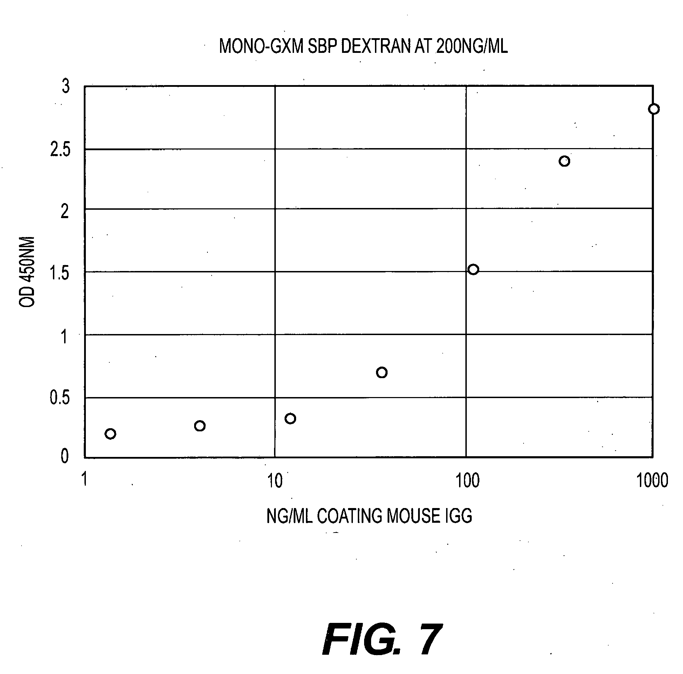

The goat-anti-mouse antibody-(SBP Dextran) polymer was diluted and incubated with a mouse IgG coated ELISA plate. After washing, the plate was developed with TMB peroxidase substrate (KPL Inc) and read using an ELISA plate reader. Results show a working 4-parameter logistic curve (see Figure 7).

Other embodiments and uses of the invention will be apparent to those skilled in the art from consideration of the specification and practice of the invention disclosed herein. All references cited herein, including all publications, U.S. and foreign patents and patent applications, are specifically and entirely incorporated by reference. The term comprising, where ever used, is intended to include the terms consisting and consisting essentially of. Furthermore, the terms comprising, including, and containing are not intended to be limiting. It is intended that the specification and examples be considered exemplary only with the true scope and spirit of the invention indicated by the following claims.

## Claims

1. A method of differentially functionalizing a polymer comprising, in any order:
functionalizing a terminal moiety of the polymer with a first functional reagent such that the polymer contains at least one functional group; and
functionalizing a non-terminal moiety of the polymer by reaction with a second functional reagent to form at least one different functional group; wherein the first functional reagent is an amino-oxy reagent.

2. A method as claimed in claim 1, wherein the terminal moiety reacted with the amino oxy reagent is a reducing end of the polymer; wherein the polymer comprises a polysaccharide, an oligosaccharide, a carbohydrate or a carbohydrate containing molecule; preferably a dextran polymer.

3. A method as claimed in claim 1 or claim 2, wherein the reaction with the second functional reagent provides a non-terminal moiety a single functional group; wherein the single functional group is an amine, a carboxyl, a thiol, hydrazide, or hydrazine group.

4. A method as claimed in any of claims 1 to 3, wherein the non-terminal moiety reacted with a functional reagent other than an amino oxy reagent is the polymer chain of a polysaccharide, an oligosaccharide, a carbohydrate or a carbohydrate containing molecule.

5. A method as claimed in any preceding claim, wherein the polymer is a dextran polymer.

6. A method as claimed in any preceding claim, wherein the reaction with the second functional reagent provides at least one non-terminal moiety a functional group which is an amine, a carboxyl, or a thiol group.

7. A method as claimed in any preceding claim, wherein the reaction with the second functional reagent provides a single non-terminal moiety a functional group.

8. A method as claimed in claim 1, wherein the reaction with the second functional group precedes functionalizing the reducing end of the polymer by reaction with an amino-oxy reagent.

9. A method as claimed in any preceding claim, wherein differentially functionalizing the polymer comprises, in any order:
functionalizing the polymer with an amino oxy reagent to react with a carbonyl group at one terminus of the polymer; and
reacting the polymer with a reagent that binds to the polymer exclusive of the terminus.

10. A method as claimed in claim 9, wherein the carbonyl group is an aldehyde.

11. A method as claimed in of claim 9 or claim 10, wherein functionalized polymer bound with the reagent is selected from the group consisting of monobiotin HRP-dextran, monoamine-carboxyl dextran, monothiol-carboxyl dextran, and monothiol amino dextran.

12. A method as claimed in any preceding claim, which is performed in an aqueous medium.

13. A differentially functionalized polymer comprising a terminal amino-oxy functionalized moiety and at least one further different non-terminal functionalized moiety.

14. A differentially functionalized polymer as claimed in claim 13, wherein the polymer comprises a structural feature or features according to any preceding claim.

15. A method as claimed in any preceding claim further comprising adding the differentially functionalized polymer to an antibody.

## Patentansprüche

1. Verfahren zum differenziellen Funktionalisieren eines Polymers, das in beliebiger Reihenfolge Folgendes umfasst:
Funktionalisieren einer endständigen Gruppierung des Polymers mit einem ersten funktionellen Reagenz derart, dass das Polymer mindestens eine funktionelle Gruppe enthält; und
Funktionalisieren einer nicht endständigen Gruppierung des Polymers durch Reaktion mit einem zweiten funktionellen Reagenz zur Bildung mindestens einer anderen funktionellen Gruppe;
wobei es sich bei dem ersten funktionellen Reagenz um ein Aminooxy-Reagenz handelt.

2. Verfahren nach Anspruch 1, bei dem es sich bei der mit dem Aminooxy-Reagenz zur Reaktion gebrachten endständigen Gruppierung um ein reduzierendes Ende des Polymers handelt, wobei das Polymer ein Polysaccharid, ein Oligosaccharid, ein Kohlenhydrat oder ein kohlenhydrathaltiges Molekül, vorzugsweise ein Dextranpolymer, umfasst.

3. Verfahren nach Anspruch 1 oder Anspruch 2, bei dem durch die Reaktion mit dem zweiten funktionellen Reagenz eine nicht endständige Gruppierung eine einzigen funktionellen Gruppe versehen wird, wobei es sich bei der einzigen funktionellen Gruppe um eine Amin-, eine Carboxyl-, eine Thiol-, eine Hydrazid- oder eine Hydrazingruppe handelt.

4. Verfahren nach einem der Ansprüche 1 bis 3, bei dem es sich bei der mit einem funktionellen Reagenz, das von einem Aminooxy-Reagenz verschieden ist, zur Reaktion gebrachten nicht endständigen Gruppierung um die Polymerkette eines Polysaccharids, eines Oligosaccharids, eines Kohlenhydrats oder eines kohlenhydrathaltigen Moleküls handelt.

5. Verfahren nach einem der vorhergehenden Ansprüche, bei dem es sich bei dem Polymer um ein Dextranpolymer handelt.

6. Verfahren nach einem der vorhergehenden Ansprüche, bei dem durch die Reaktion mit dem zweiten funktionellen Reagenz mindestens eine nicht endständige Gruppierung eine funktionellen Gruppe versehen wird, bei der es sich um um eine Amin-, eine Carboxyl- oder eine Thiolgruppe handelt.

7. Verfahren nach einem der vorhergehenden Ansprüche, bei dem durch die Reaktion mit dem zweiten funktionellen Reagenz eine einzige nicht endständige Gruppierung eine funktionellen Gruppe versehen wird.

8. Verfahren nach Anspruch 1, bei dem die Reaktion mit der zweiten funktionellen Gruppe vor dem Funktionalisieren des reduzierenden Endes des Polymers durch Reaktion mit einem Aminooxy-Reagenz erfolgt.

9. Verfahren nach einem der vorhergehenden Ansprüche, wobei das differenzielle Funktionalisieren des Polymers in beliebiger Reihenfolge Folgendes umfasst:
Funktionalisieren des Polymers mit einem Aminooxy-Reagenz zur Reaktion mit einer Carbonylgruppe an einem Terminus des Polymers; und
Umsetzen des Polymers mit einem Reagenz, das an das Polymer ausschließlich des Terminus bindet.

10. Verfahren nach Anspruch 9, bei dem es sich bei der Carbonylgruppe um einen Aldehyd handelt.

11. Verfahren nach Anspruch 9 oder Anspruch 10, bei dem das mit dem Reagenz gebundene funktionalisierte Polymer aus der Gruppe bestehend aus Monobiotin-HRP-dextran, Monoamincarboxyldextran, Monothiolcarboxyldextran und Monothiolaminodextran ausgewählt wird.

12. Verfahren nach einem der vorhergehenden Ansprüche, das in einem wässrigen Medium durchgeführt wird.

13. Differenziell funktionalisiertes Polymer mit einer endständigen aminooxyfunktionalisierten Gruppierung und mindestens einer weiteren anderen nicht endständigen funktionalisierten Gruppierung.

14. Differenziell funktionalisiertes Polymer nach Anspruch 13, wobei das Polymer ein Strukturmerkmal bzw. Strukturmerkmale gemäß einem der vorhergehenden Ansprüche umfasst.

15. Verfahren nach einem der vorhergehenden Ansprüche, ferner umfassend das Addieren des differenziell funktionalisierten Polymers an einen Antikörper.

## Revendications

1. Méthode de fonctionnalisation de manière différentielle d'un polymère comprenant, dans un ordre quelconque :
la fonctionnalisation d'un motif terminal du polymère par un premier réactif fonctionnel de façon à ce que le polymère contienne au moins un groupement fonctionnel ; et
la fonctionnalisation d'un motif non terminal du polymère par réaction avec un deuxième réactif fonctionnel afin de former au moins un groupement fonctionnel différent ; où le premier réactif fonctionnel est un réactif aminooxy.

2. Méthode selon la revendication 1, dans laquelle le motif terminal réagi avec le réactif aminooxy est une extrémité réductrice du polymère ; où le polymère comprend un polysaccharide, un oligosaccharide, un glucide ou une molécule contenant un glucide ; préférablement un polymère de dextrane.

3. Méthode selon la revendication 1 ou la revendication 2, dans laquelle la réaction avec le deuxième réactif fonctionnel fournit un motif non terminal un groupement fonctionnel unique ; où le groupement fonctionnel unique est un groupement amine, carboxyle, thiol, hydrazide ou hydrazine.

4. Méthode selon l'une quelconque des revendications 1 à 3, dans laquelle le motif non terminal réagi avec un réactif fonctionnel autre qu'un réactif aminooxy est la chaîne de polymère d'un polysaccharide, d'un oligosaccharide, d'un glucide ou d'une molécule contenant un glucide.

5. Méthode selon l'une quelconque des revendications précédentes, dans laquelle le polymère est un polymère de dextrane.

6. Méthode selon l'une quelconque des revendications précédentes, dans laquelle la réaction avec le deuxième réactif fonctionnel fournit au moins un motif non terminal un groupement fonctionnel qui est un groupement amine, carboxyle ou thiol.

7. Méthode selon l'une quelconque des revendications précédentes, dans laquelle la réaction avec le deuxième réactif fonctionnel fournit un motif non terminal unique un groupement fonctionnel.

8. Méthode selon la revendication 1, dans laquelle la réaction avec le deuxième groupement fonctionnel précède la fonctionnalisation de l'extrémité réductrice du polymère par réaction avec un réactif aminooxy.

9. Méthode selon l'une quelconque des revendications précédentes, dans laquelle la fonctionnalisation de manière différentielle du polymère comprend, dans un ordre quelconque :
la fonctionnalisation du polymère par un réactif aminooxy afin de réagir avec un groupement carbonyle au niveau d'une extrémité terminale du polymère ; et
la réaction du polymère avec un réactif qui se fixe au polymère, excepté l'extrémité terminale.

10. Méthode selon la revendication 9, dans laquelle le groupement carbonyle est un aldéhyde.

11. Méthode selon la revendication 9 ou la revendication 10, dans laquelle le polymère fonctionnalisé fixé par le réactif est choisi dans le groupe constitué par la mono-biotine HRP-dextrane, la monoamine-carboxydextrane, le monothiol-carboxydextrane, et le monothiol-aminodextrane.

12. Méthode selon l'une quelconque des revendications précédentes, qui est effectuée dans un milieu aqueux.

13. Polymère fonctionnalisé de manière différentielle, comprenant un motif fonctionnalisé aminooxy terminal et au moins un autre motif fonctionnalisé non terminal différent.

14. Polymère fonctionnalisé de manière différentielle selon la revendication 13, dans lequel le polymère comprend une ou plusieurs caractéristiques structurelles selon l'une quelconque des revendications précédentes.

15. Méthode selon l'une quelconque des revendications précédentes, comprenant en outre l'addition du polymère fonctionnalisé de manière différentielle à un anticorps.
